(19) Europäisches Patentamt
European Patent Office
Office européen des brevets

(11) Veröffentlichungsnummer: **0 125 406**
A2

(12) # EUROPÄISCHE PATENTANMELDUNG

(21) Anmeldenummer: 84102283.3

(22) Anmeldetag: 03.03.84

(51) Int. Cl.³: **A 61 K 31/415**
//C07D231/00

(30) Priorität: 12.03.83 DE 3308881
25.11.83 DE 3342628

(43) Veröffentlichungstag der Anmeldung:
21.11.84 Patentblatt 84/47

(84) Benannte Vertragsstaaten:
AT BE CH DE FR GB IT LI LU NL SE

(71) Anmelder: BAYER AG
Konzernverwaltung RP Patentabteilung
D-5090 Leverkusen 1 Bayerwerk(DE)

(72) Erfinder: Mardin, Mithat, Dr.
400 Morgan Lane
West Haven, Ct. 06516(US)

(72) Erfinder: Seuter, Friedel, Dr.
Moospfad 16
D-5600 Wuppertal 1(DE)

(72) Erfinder: Perzborn, Elisabeth, Dr.
Am Tescher Busch 13
D-5600 Wuppertal 11(DE)

(72) Erfinder: Schlossmann, Klaus, Dr.
In der Beek 116
D-5600 Wuppertal 1(DE)

(72) Erfinder: Mayer, Dieter, Prof. Dr.
Beethovenstrasse 1
D-4712 Werne(DE)

(72) Erfinder: Fiedler, Volker, Dr.
Kluser Hoehe 6
D-5600 Wuppertal 1(DE)

(54) **Neue therapeutische Verwendung von Pyrazolonderivaten, Arzneimittel hierfür und Verfahren zu deren Herstellung.**

(57) Die vorliegende Erfindung betrifft die Verwendung bestimmter 1-substituierter Pyrazolon-(5)-Derivate als Lipoxygenasehemmer, zur Verhütung und Behandlung von Ischämien, insbesondere von Herzinfarkten nach Myokardischämien, von Herzrhythmusstörungen, von Hautentzündungen (insbesondere Psoriasis) und Augenentzündungen, von rheumatischen, allergischen und asthmatischen Erkrankungen, Schocklunge, Lungenembolien und Oedemen (insbesondere Lungenoedemen), von pulmonaler Hypertension, Sauerstoffintoxikationen sowie von Ulcerationen. Gegenstand der Erfindung sind auch Arzneimittel gegen Haut- und Augenentzündungen und Schocklunge sowie lipoxygenasehemmende, bronchodilatorische, antiarrhythmische, antiischämische, antirheumetische, anti-

EP 0 125 406 A2

Croydon Printing Company Ltd

0125406

BAYER AKTIENGESELLSCHAFT 5090 Leverkusen, Bayerwerk

Konzernverwaltung RP
Patentabteilung Sft/bc/c

BEZEICHNUNG GEÄNDERT
siehe Titelseite

Verwendung von Pyrazolonderivaten als Lipoxygenasehemmer, bei der Therapie von Ischämien und Herzrhythmusstörungen, Haut- und Augenentzündungen, sowie von rheumatischen, allergischen und asthmatischen Erkrankungen, Oedemen, Lungenembolien, pulmonaler Hypertension, Schocklunge, Sauerstoffintoxikation und Ulcerationen, Arzneimittel hierfür und Verfahren zu deren Herstellung

Die vorliegende Erfindung betrifft die Verwendung bestimmter 1-substituierter Pyrazolon-(5)-Derivate als Lipoxygenasehemmer, zur Verhütung und Behandlung von Ischämien, insbesondere von Herzinfarkten nach Myokardischämien, von Herzrhythmusstörungen, von Hautentzündungen (insbesondere Psoriasis) und Augenentzündungen, von rheumatischen, allergischen und asthmatischen Erkrankungen, Schocklunge, Lungenembolien und Oedemen (insbesondere Lungenoedemen), von pulmonaler Hypertension, Sauerstoffintoxikationen sowie von Ulcerationen. Gegenstand der Erfindung sind auch Arzneimittel gegen Haut- und Augenentzündungen und Schocklunge sowie lipoxygenasehemmende, bronchodilatorische, antiarrhythmische, antiischämische, antirheumatische, antiallergische, antiasthmatische, antioedemische und gastroprotektive Arzneimittel, welche durch den Gehalt an einer therapeutisch wirksamen Menge des Pyrazolonderivats gekennzeichnet sind.

In US-PS 3 147 276 wird beschrieben, daß gewisse 1-substituierte Pyrazolone-(5) antiinflammatorische Eigenschaften besitzen. Weiter ist bekannt, daß zahlreiche Pyrazolon-(5)-Derivate als Diuretika, Antihypertensiva und Antithrombotika eingesetzt werden können (DE-OS 2 319 280, DE-OS 2 554 701, DE-OS 2 363 511 und DE-OS 2 554 703).

Als mit Abstand stärkstes Antithrombotikum erwies sich das 3-Methyl-1-/2-(2-naphthyloxy)-ethyl7-2-pyrazolin-5-on ("Nafazatrom"), das in DE-AS 2 247 272 (US-PS 4 053 621) beschrieben wird. Von Nafazatrom ist auch bekannt geworden, daß es als Stimulator von endogenem Prostacyclin ($PGI_2$) aus dem Gefäßendothel wirken kann (Vermylen et al., Lancet I, 528, 1979, Chamone et al., Haemostasis 10, 297, 1981, McIntyre und Salzman, Thrombosis and Haemostasis, 46, Abstr. No. 45, 1981). Außerdem wurde von Wong und McGiff (J. Pharmacol. Exp. Ther. 223, 757-760, 1982) gezeigt, daß Nafazatrom die 15-Hydroxyprostaglandindehydrogenase, das $PGI_2$-abbauende Enzym, blockiert.

Darüber hinaus hemmt die Substanz die Lipoxygenaseaktivität (Busse et al., Fed. Proc. 41, 1717, 1982).

Wie sich zeigte, ist die antithrombotische Wirkung von Nafazatrom außerordentlich strukturabhängig: So vermindert beispielsweise die Substitution von Methyl gegen Ethyl in der 3-Position des Nafazatroms dessen Wirkung bei der Thromboseprophylaxe auf etwa 1/30, während beim entsprechenden 3-Benzyl-, 3-Isopropyl-, 3-n-Propyl- sowie 3,4-Dimethyl-Derivat auch bei 100-facher Dosissteigerung gegenüber Nafazatrom kein Effekt feststellbar ist. Es ist daher als äußerst überraschend anzusehen, daß diese Verbindungen und auch strukturell noch fernerliegende Pyrazolonderivate dem Nafazatrom hinsichtlich der anderen oben erwähnten Wirkqualitäten zumindest gleichwertig sind.

Die erfindungsgemäß einzusetzenden Pyrazolonderivate entsprechen der allgemeinen Formel

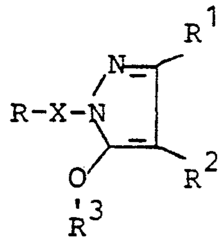

(I)

worin

R für einen Aryl- oder Heteroarylrest mit 6 bis 12 C-Atomen steht, der gegebenenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, $SO_n$-Alkyl (n = 0 bis 2) oder $SO_n$-Trifluormethyl (n = 0 bis 2) enthält, wobei unter Alkyl, Alkenyl und Alkoxy jeweils Reste mit 1 bis 4 C-Atomen zu verstehen sind,

$R^1$ Wasserstoff oder einen Kohlenwasserstoffrest mit 1 bis 18, vorzugsweise 1 bis 8 C-Atomen, welcher gegebenenfalls eine Hydroxy-, Ether-, Ester- oder Carboxylgruppe sowie gegebenenfalls 1 bis 3 Halogenatome enthält, darstellt,

$R^2$ die Bedeutung von $R^1$ hat oder für einen Rest

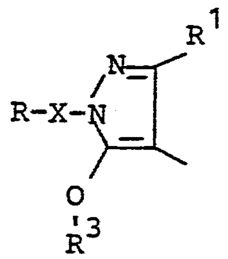

steht,

$R^3$ Wasserstoff, einen Acyl- oder Sulfonylrest mit jeweils 1 bis 15 C-Atomen darstellt und

X für eine der Gruppen

$-CH_2-CH_2-CH_2-$, $-O-CH_2-CH_2-$, $-S-CH_2-CH_2-$, $-CH_2-CH_2-$, $-CH_2-$ oder $-CH(CH_3)-$

steht, wobei das Sauerstoff- bzw. Schwefelatom an den Rest R gebunden ist, mit der Maßgabe, daß X nicht für $-O-CH_2-CH_2-$ steht, wenn R = ß-Naphthyl; $R^1 = CH_3$ und $R^2 = R^3 = H$.

Erfindungsgemäß bevorzugt einzusetzende Verbindungen der allgemeinen Formel (I) sind solche, bei denen

R für einen gegebenenfalls substituierten Naphthyl-, Diphenyl- oder Phenylrest steht, besonders bevorzugt einen Naphthyl- oder Diphenylrest, insbesondere

α- oder ß-Naphthyl. Bevorzugt tragen diese Reste 0, 1 oder 2 Substituenten, insbesondere Br oder Cl. Bromnaphthylreste sind besonders bevorzugt.

Erfindungsgemäß werden weiterhin solche Verbindungen der allgemeinen Formel (I) bevorzugt, bei denen

$R^1$ für einen Phenyl-, Benzyl- oder Cyclohexylrest oder einen aliphatischen Kohlenwasserstoffrest, vorzugsweise einen Rest mit 1 bis 4 C-Atomen, welcher gegebenenfalls eine Hydroxyl-, Ether- oder Estergruppe aufweist, besonders bevorzugt eine gegebenenfalls verzweigte $C_1$-$C_4$-Alkylgruppe steht, und

$R^2$ die bevorzugte Bedeutung von $R^1$ besitzt oder auch Wasserstoff darstellt. Besonders bevorzugt ist mindestens einer der Reste $R^1$ oder $R^2$ nicht Wasserstoff und $R^1$ und $R^2$ besitzen zusammen 2 bis 7, insbesondere 2 bis 4 C-Atome.

Weiterhin sind erfindungsgemäß Verbindungen der allgemeinen Formel (I) bevorzugt, bei denen

$R^3$ für Wasserstoff oder einen aromatischen Acylrest mit 7 bis 13 C-Atomen, insbesondere einen Carboxyphenyl- oder Carboxynaphthylrest steht, der gegebenenfalls 1 oder 2 Substituenten aus der Gruppe Halogen, Trifluormethyl, $C_1$-$C_4$-Alkoxy und Nitro enthält. Besonders bevorzugt ist $R^3$ Wasserstoff.

Erfindungsgemäß bevorzugt sind schließlich auch Verbindungen, bei welchen X für $-O-CH_2-CH_2-$, $-S-CH_2-CH_2-$, $-CH_2-CH_2-$, $-CH_2-CH_2-CH_2-$ oder $-\underset{CH_3}{\underset{|}{CH}}-$ steht, insbesondere für $-O-CH_2-CH_2-$, $-CH_2-CH_2-CH_2-$ oder $-\underset{CH_3}{\underset{|}{CH}}-$ .

Wenn $R^3 = H$, dann können die erfindungsgemäß einzusetzenden Verbindungen außer in der durch Formel (I) repräsentierten Form auch in einer der folgenden tautomeren Formen oder als Gemisch der möglichen tautomeren Formen vorliegen:

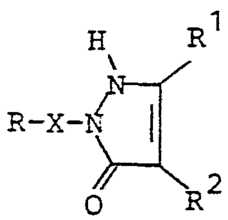 (Ia) 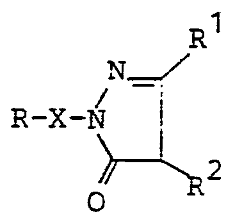 (Ib)

Die erfindungsgemäß einzusetzenden Pyrazolonderivate der allgemeinen Formel (I) sind aus DE-OS 2 319 280 (US-PS 3 957 814), DE-OS 2 363 511 (US-PS 4 002 641), DE-OS 2 554 701 und DE-OS 2 554 703 bekannt und können nach den dort im Detail beschriebenen Verfahren hergestellt werden.

Verbindungen der Formel (I), bei welchen $R^3 = H$, werden erhalten, wenn man

A) Hydrazine der Formel

$$R-X-NH-NH_2 \qquad (II)$$

in welcher
R und X die oben angegebene Bedeutung haben,

mit ß-Ketosäurederivaten der Formel

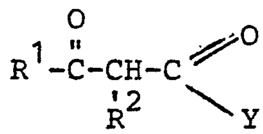

(III)

in welcher

$R^1$ und $R^2$ die oben angegebene Bedeutung haben und
Y für einen austretenden Rest, z.B. einen Hydroxy-, Alkoxy-, Aralkoxy-, Amino- oder Alkylaminorest steht,

gegebenenfalls in Gegenwart inerter Lösungsmittel und basischer oder saurer Katalysatoren wie Alkali- und Erdalkalihydroxide und -carbonate oder wie Halogenwasserstoffsäuren, Schwefelsäure oder Sulfonsäuren, bei Temperaturen zwischen 10 und 200°C umsetzt,

oder

B) Verbindungen der Formel

R-X-A (IV)

in welcher
R die oben angegebene Bedeutung hat und

A einen austretenden Rest wie Halogen oder einen Dialkyloxonium-, Dialkylsulfonium- oder Trialkylammoniumrest oder einen Aryl- oder Trifluormethylsulfonsäurerest darstellt,

mit Pyrazolon-(5)-derivaten der Formel

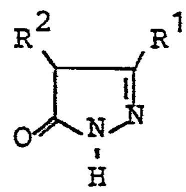

(V)

in welcher
$R^1$ und $R^2$ die oben angegebene Bedeutung haben,

gegebenenfalls in Gegenwart inerter Lösungsmittel und anorganischer oder organischer Basen wie Alkalihydroxide, -carbonate, -alkoholate, -hydride oder -amide bei Temperaturen zwischen 10 und 200°C umsetzt,

oder

C) Acetylencarbonsäurederivate der Formel

$$Y-\overset{O}{\overset{\|}{C}}-C\equiv C-R^1 \qquad (VI)$$

in welcher
$R^1$ und Y die oben angegebene Bedeutung haben,

mit Hydrazinen der obigen Formel (II), gegebenenfalls in Gegenwart inerter Lösungsmittel und anorganischer oder organischer Basen, bei Temperaturen zwischen 50 und 200°C umsetzt.

Verbindungen der Formel (I), in denen $R^3 \neq H$, werden erhalten, indem man eine Verbindung der Formel

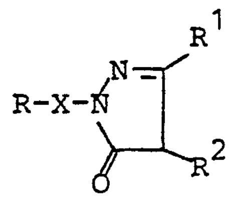

in welcher
R, X, $R^1$ und $R^2$ die oben angegebene Bedeutung haben,

mit Säurederivaten, vorzugsweise mit

a) Carbon- bzw. Kohlensäurederivaten der Formel

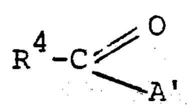
(VII)

in welcher

A' einen austretenden Rest wie Halogen oder einen 5-gliedrigen, heterocyclischen Azolring oder eine über ein Sauerstoff- oder Schwefelatom an den Carbonyl-Kohlenstoff gebundene Alkylgruppe oder einen gegebenenfalls durch 1 bis 2 Nitrogruppen substituierten Phenylrest oder einen Acyloxyrest bedeutet und

$R^4$-C=O die Bedeutung von $R^3$ hat,

oder

b) mit Sulfonsäurederivaten der Formel

$$R^5\text{-}SO_2\text{-}A'' \qquad (VIII)$$

in welcher

$R^5$-$SO_2$ die Bedeutung von $R^3$ hat und

A" für Halogen steht,

gegebenenfalls in Gegenwart inerter Lösungsmittel und basischer Hilfsstoffe, wie Alkali- oder Erdalkalihydroxide und -carbonate oder organischer Basen, wie Triethylamin oder Pyridin bei Temperaturen zwischen -20 und 150°C umsetzt.

Die erfindungsgemäß einzusetzenden Pyrazolonderivate hemmen überraschenderweise die Lipoxygenaseaktivität, welche für die Biosynthese des chemotaktisch wirksamen Leukotriens $B_4$ und der spasmogen wirksamen Leukotriene $C_4$ und $D_4$ verantwortlich ist.

Bekannte Lipoxygenasehemmer wie Nordihydroguaretsäure, 3-Amino-1-(3-trifluormethylphenyl)-pyrazolin (BW 755 C), Phenidon und 5,8,11,14-Eikosatetransäure haben den Nachteil, daß sie entweder gleichzeitig als Cyclooxygenasehemmer oder erst bei sehr hohen Konzentrationen wirksam sind. Die Hemmung des Enzyms Cyclooxygenase des Arachidonsäuremetabolismus führt zu einer globalen Prostaglandinsynthesehemmung und zu einer Stimulation des Lipoxygenaseweges, was Gastrotoxizität, entzündungsfördernde und asthmatische Wirkungen und erhöhte Thromboseneigung infolge Hemmung der Prostacyclinsynthese verursachen kann.

Eine derartige Cyclooxygenasehemmung tritt bei der Applikation der erfindungsgemäß zu verwendenden Pyrazolonderivate nicht ein. Diese zeigen im Gegenteil eine ausgeprägte antiasthmatische, entzündungshemmende und gastroprotektive Wirkung.

Überraschenderweise hemmen die erfindungsgemäß zu verwendenden Pyrazolonderivate selektiv die für die Biosynthese der Leukotriene $B_4$, $C_4$ und $D_4$ verantwortliche 5-Lipoxygenase, während die für die Biosynthese des endogenen Lipoxygenaseinhibitors 15-Hydroxyeikosatetraensäure (15-HETE) zuständige 15-Lipoxygenase nicht gehemmt bzw. bei Konzentrationen 10µg/ml sogar stimuliert wird. Ebenso wird die 12-Lipoxygenaseaktivität durch die erfindungsgemäß zu verwendenden Pyrazolonderivate bei diesen Konzentrationen nicht gehemmt. Außerdem stimulieren die erfindungsgemäß zu verwendenden Pyrazolonderivate spezifisch die Synthese von Prostacyclin ($PGI_2$).

Überraschenderweise wird in Gegenwart der erfindungsgemäß einzusetzenden Pyrazolonderivate nur die $PGI_2$-Synthese stimuliert, während die Synthese der vasokonstriktorisch wirksamen Prostaglandine und des $TXA_2$ nicht beeinflußt ist. Die erfindungsgemäß einzusetzenden Pyrazolonderivate weisen auch lokal gefäßdilatierende Effekte

auf, die besonders im ischämischen Gewebe (z.B. Herz, Hirn, Peripherie) von therapeutischem Nutzen sind. Im Unterschied zu Indomethacin, welches die Prostaglandinsynthese aus der Arachidonsäure insgesamt hemmt, greifen die erfindungsgemäß einzusetzenden Pyrazolonderivate viel spezifischer in den Stoffwechsel der für die Bildung des $PGI_2$, des Thromboxans und der Leukotriene maßgebenden Enzyme ein, so daß nicht nur der schädigende, gefäßverengende und arrhythmienerhöhende Einfluß des Thromboxans und der Leukotriene reduziert sondern auch der gefäßerweiternde Einfluß des Prostacyclins durch Stimulierung seiner Bildung erhöht wird.

Hinzu kommt, daß die erfindungsgemäß einzusetzenden Pyrazolonderivate die schädigende Wirkung eines plötzlichen Sauerstoffeinstroms nach einer hypoxischen oder anoxischen Periode reduzieren bzw. ausschalten. Schädigungen durch Reoxygenierung können nach Bypass-Operationen auftreten. Die erfindungsgemäß einzusetzenden Pyrazolonderivate werden daher bei derartigen Operationen geeignet sein, Sauerstoffschäden zu verhindern.

Überraschenderweise wurde auch gefunden, daß die erfindungsgemäß einzusetzenden Pyrazolonderivate die Klebrigkeit von Leukozyten (stickiness) vermindern, d.h. deren Wanderungsgeschwindigkeit erhöhen. Diese Verminderung der Leukozytenklebrigkeit trägt ebenfalls zur Abnahme ischämischer Gebiete bei, denn die Leukozyten passieren nach Behandlung mit den erfindungsgemäß einzusetzenden Pyrazolonderivaten den mikrozirkulatorischen Bereich dieser Gebiete, ohne durch totalen oder zeitweiligen Verschluß von kleinen Gefäßen bzw. Kapillaren eine Minderversorgung des entsprechenden Gewebes hervorzurufen.

Die erfindungsgemäß einzusetzenden Pyrazolonderivate können darüber hinaus auch zur Therapie von pulmonaler Hypertension, Lungenembolien, Lungenoedemen und Schocklunge (ARDS = "Adult Respiratory Distress Syndrom") herangezogen werden. Die bronchodilatierenden Eigenschaften der Wirkstoffe lassen sich z.B. an der isolierten Meerschweinchenlunge (nach der Methode von F.P. Luduena et al., Arch.Int.Pharmacodyn. 111, 392, 1957) zeigen.

Die erfindungsgemäß einzusetzenden Verbindungen eignen sich auch für die Therapie von Augen- und Hautentzündungen, insbesondere Psoriasis und Vitiligo.

Die erfindungsgemäß einzusetzenden Pyrazolonderivate vermindern darüberhinaus überraschenderweise die Größe von Herzinfarkten nach Myokardischämien und besitzen positiven Einfluß auf Herzrhythmusstörungen. Auch diese erfindungsgemäßen Verwendungszwecke der erfindungsgemäß einzusetzenden Pyrazolonderivate werden durch die eingangs beschriebenen, aus dem Stand der Technik bekannten Indikationen nicht nahegelegt:

Substanzen zur Therapie von Herzrhythmusstörungen werden nach ihren elektrophysiologischen Wirkungen eingeteilt.

Seit der Klassifizierung von Vaughan Williams (Pharmac. Ther. B 1, 115, 1975) unterscheidet man folgende Klassen:

I) Membranstabilisierende Antiarrhythmika vom
   a) Chinidintyp, z.B. Procain, Ajmalin
   b) Lidocaintyp, z.B. Lidocain, Diphenylhydantoin

II) Beta-Rezeptorenblocker, wie z.B. Propanolol u.v.a.

III) Calciumantagonisten, wie z.B. Verapamil, Nifedipin u.v.a.

IV) Substanzen mit Verlängerung der Aktionspotentialdauer, wie z.B. Amiodaron.

Die erfindungsgemäß einzusetzenden Pyrazolonderivate sind weder aufgrund ihrer chemischen Struktur noch ihrer bisher bekannten Wirkungen unter die obigen Gruppen zu subsumieren. Es war aus diesem Grund nicht vorhersehbar, daß die erfindungsgemäß einzusetzenden Pyrazolonderivate antiarrhythmische Effekte entfalten. Ebensowenig war aufgrund der antithrombotischen Wirkung zu erwarten, daß die erfindungsgemäß einzusetzenden Pyrazolonderivate nach erfolgtem Gefäßverschluß - und zwar unabhängig von der Natur des Koranargefäßverschlusses - die Ausbreitung der Myokardischämie stark einschränken und damit die Infarktgröße vermindern und darüber hinaus zu einer rascheren Heilung und geringeren Kreislaufnebenwirkungen führen. Wie durch Tierversuche gezeigt werden kann, verringern die erfindungsgemäß

einzusetzenden Pyrazolonderivate die Ischämie-bedingte Anhebung der ST-Strecke als Infarktzeichen; darüber hinaus steigt die R-Welle der peripheren Extremitäten-EKG weniger stark an und bildet sich schneller zurück; ST-Intervalle werden weniger als in den Kontrollen verändert, was auf eine verbesserte Repolarisation der Herzmuskelzellen bei vermindertem Herzinfarkt schließen läßt.

In den Arzneimitteln gemäß vorliegender Erfindung sind neben den erfindungsgemäß einzusetzenden Pyrazolonderivaten auch pharmazeutisch unbedenkliche Verdünnungsmittel oder Trägermaterialien enthalten. Hierunter sind nicht-toxische Substanzen zu verstehen, die nach dem Vermischen mit dem Wirkstoff diesen in einer für die Verabreichung geeigneten Form liefern. Die Bezeichnung schließt vorzugsweise Wasser und üblicherweise bei der chemischen Synthese verwendete organische Lösungsmittel mit niederem Molekulargewicht aus, außer bei Vorliegen anderer pharmazeutisch erforderlicher Bestandteile wie Salze in den richtigen Mengen, um eine isotonische Zubereitung herzustellen, Puffer, oberflächenaktive Mittel, Farb- und Geschmacksstoffe und Konservierungsmittel. Beispiele für geeignete feste und flüssige Verdünnungsmittel und Trägermaterialien sind die folgenden: Wasserhaltige Puffermittel, die durch Zusatz von Glucose oder Salzen isotonisch gemacht werden können; nicht-toxische organische Lösungsmittel wie Paraffine, pflanzliche Öle, Alkohole, Glykole; gemahlene Natursteinmaterialien (beispielsweise Kaoline, Aluminium-

0125406

oxide, Talkum oder Kreide); synthetische Gesteinspulver (beispielsweise hochdisperse Kieselsäure oder Silikate); Zucker; und wäßrige Suspensionen von Cellulosederivaten, z.B. Methylhydroxyethylcellulose (Tylose).

In der Regel enthalten die erfindungsgemäßen Arzneimittel 0,5 bis 95 Gew.-%, bevorzugt 1 bis 90 Gew.-%, besonders bevorzugt 5 bis 50 Gew.-%, an den erfindungsgemäß einzusetzenden Pyrazolonderivaten.

Die orale Verabreichung kann unter Verwendung fester und flüssiger Dosierungsformen wie Pulver, Tabletten, Dragees, Kapseln, Granulat, Suspensionen, Lösungen und dergleichen vorgenommen werden. Gegebenenfalls können die Dosierungseinheiten für die orale Verabreichung mikroverkapselt werden, um die Abgabe hinauszuzögern oder über längere Zeit hinweg zu verlangsamen, wie beispielsweise durch Überziehen oder Einbetten von teilchenförmigem Material in Polymere, Wachs oder dergleichen.

Die parenterale Verabreichung kann unter Verwendung flüssiger Dosierungsformen wie steriler Lösungen und Suspensionen erfolgen, die für die subkutane, intramuskuläre oder intravenöse Injektion bestimmt sind. Diese werden durch Suspendieren oder Auflösen einer abgemessenen Menge des Wirkstoffes in einem zur Injektion geeigneten nicht-toxischen flüssigen Streckmittel wie einem wäßrigen oder öligen Medium und Sterilisierung der Suspension oder Lösung hergestellt. Stabilisatoren, Konservierungsmittel und Emulgatoren können ebenfalls zugesetzt werden.

Im allgemeinen beträgt beim Menschen die auf das Körpergewicht bezogene Tagesdosis des Wirkstoffs für die parenterale Verabreichung 0,01 bis 50 mg/kg, vorzugsweise 0,1 bis 10 mg/kg, und für die orale Verabreichung 0,1 bis 500 mg/kg, vorzugsweise 0,5 bis 100 mg/kg, besonders bevorzugt 1 bis 50 mg/kg.

Die Dosierungseinheit (Tablette, Kapsel, etc.) entält in der Regel 1 bis 100 mg, bevorzugt 5 bis 50 mg, besonders bevorzugt 10 bis 30 mg, an den erfindungsgemäß einzusetzenden Pyrazolonderivaten.

Beispiel 1

Lipoxygenasehemmende Wirkung

Die im vorliegenden Beispiel verwendeten Human-PMN-Leukozyten (   95 %) wurden aus heparinisiertem Vollblut durch eine Dextran-Sedimentation und anschließende Dichtegradiententrennung (Ficoll-Paque) gewonnen (vgl. A. Boyum, Scand.J.Immunol., 5, Suppl. 5, 9, 1976).

2 x $10^7$ Zellen/ml wurden in $Ca^{2+}$-haltigem Dulbecco-Phosphat-Puffer suspendiert und in Anwesenheit bzw. Abwesenheit von Lipoxygenasehemmer mit radioaktiv markierter Arachidonsäure und dem Calcium-Ionophor A 23187 inkubiert. Nach 15 Minuten wurden die markierten Lipoxygenaseprodukte aus dem angesäuerten Inkubationsmedium extrahiert und mit einem für die Leukotriene (5-HETE-$LTB_4$) geeigneten Fließmittelgemisch dünnschichtchromatographisch getrennt (vgl. B. Jakschik et al., Biochem.Biophys.Res.Commun., 102, 624, 1981).

Die Verteilung der Aktivität auf die verschiedenen Metaboliten wurde am Dünnschichtscanner gemessen. Sie bildet ein Maß für die Lipoxygenasehemmwirkung einer Prüfsubstanz bei einer bestimmten Konzentration.

Unabhängig wurden die Lipoxygenasemetaboliten auch noch mittels HPLC bestimmt. Diese Methode erlaubt es, mit Hilfe der UV-Detektion der Leukotriene den endogenen Arachidonsäuremetabolismus zu studieren. Hierbei wurden die Zellen wie oben beschrieben behandelt mit der Ausnahme, daß keine radioaktiv markierte Arachidonsäure exogen hinzugefügt wurde. Die Hochdruckflüssigkeitschromatographie wurde auf Lichrosorb RP-18(5µm)-Säulen durchgeführt. Das Laufmittel war Methanol/Wasser/Eisessig 69/31/0.01. Die Flußrate betrug 1 ml/min. Leukotrien $B_4$ wurde bei 280 nm, die HETE-Derivate bei 232 nm mittels der UV-Absorption bestimmt.

Die Hemmung der $LTB_4$-Biosynthese durch verschiedene Pyrazolonderivate zeigt die nachstehende Tabelle 1:

Tabelle 1

| Verbindung | Konzentration (g/ml) | Hemmung (%) |
| --- | --- | --- |
| 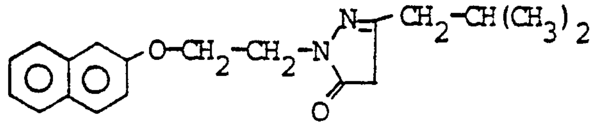 | $10^{-5}$<br>$5.10^{-6}$<br>$10^{-6}$<br>$5.10^{-7}$ | 100<br>100<br>100<br>89 |
| 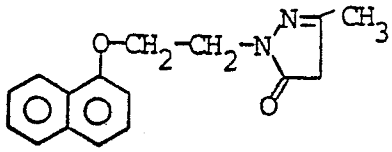 | $10^{-5}$<br>$5.10^{-6}$<br>$10^{-6}$<br>$5.10^{-7}$ | 100<br>87<br>62<br>30 |
| 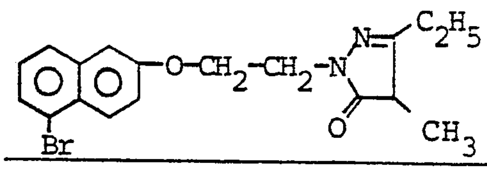 | $10^{-5}$<br>$5.10^{-6}$<br>$10^{-6}$ | 100<br>100<br>100 |
| 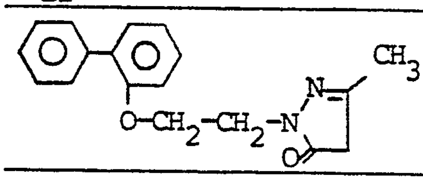 | $10^{-5}$<br>$5.10^{-6}$<br>$10^{-6}$<br>$5.10^{-7}$ | 100<br>100<br>75<br>63 |
| 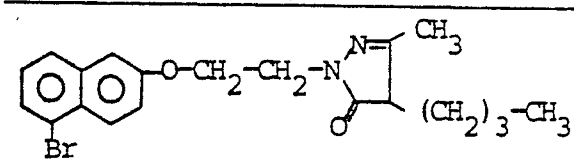 | $10^{-5}$<br>$5.10^{-6}$<br>$10^{-6}$<br>$5.10^{-7}$ | 100<br>87<br>68<br>31 |
| 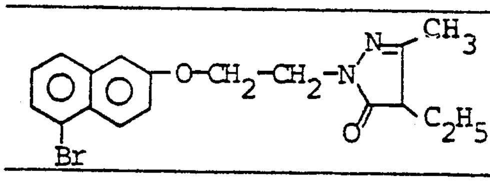 | $10^{-5}$<br>$5.10^{-6}$<br>$10^{-6}$<br>$5.10^{-7}$ | 100<br>100<br>100<br>82 |
| 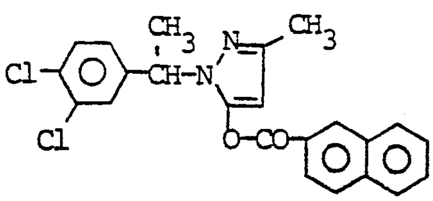 | $10^{-5}$ | 46 |
| 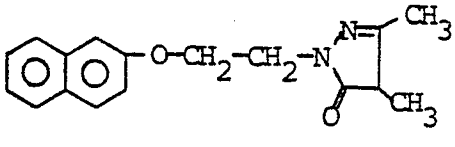 | $10^{-5}$<br>$5.10^{-6}$<br>$10^{-6}$<br>$5.10^{-7}$ | 100<br>100<br>83<br>75 |

Tabelle 1 (Fortsetzung)

| Verbindung | Konzentration (g/ml) | Hemmung (%) |
| --- | --- | --- |
| 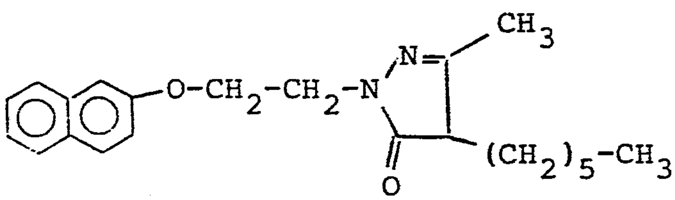 | $10^{-5}$<br>$5.10^{-5}$<br>$10^{-6}$ | 100<br>100<br>93 |
| 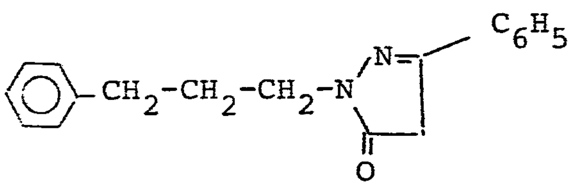 | $10^{-5}$<br>$5.10^{-6}$<br>$10^{-6}$ | 100<br>100<br>81 |
| 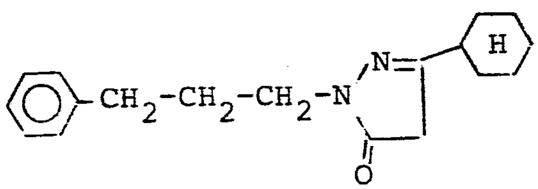 | $10^{-5}$<br>$5.10^{-6}$<br>$10^{-6}$ | 100<br>92<br>71 |
| 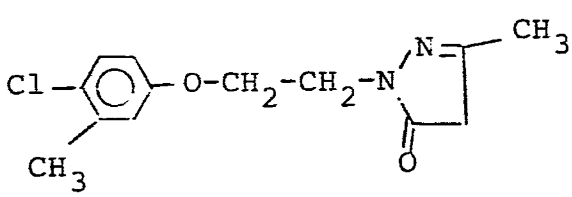 | $10^{-5}$<br>$5.10^{-6}$<br>$10^{-6}$ | 100<br>92<br>43 |
| 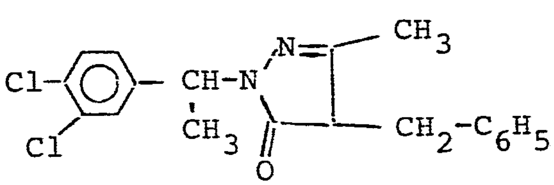 | $10^{-5}$<br>$5.10^{-6}$ | 100<br>100 |

Tabelle 1 (Fortsetzung)

| Verbindung | Konzentration (g/ml) | Hemmung (%) |
|---|---|---|
| 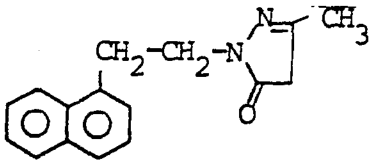 | $10^{-5}$<br>$5.10^{-6}$<br>$10^{-6}$ | 100<br>75<br>58 |
| 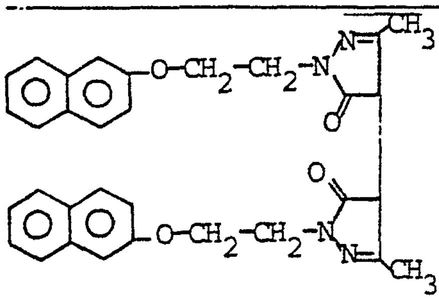 | $10^{-5}$ | 50 |
| 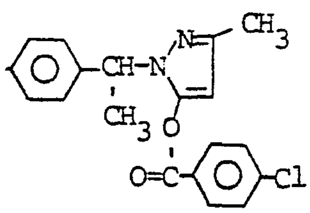 | $10^{-5}$<br>$5.10^{-6}$ | 100<br>80 |
| 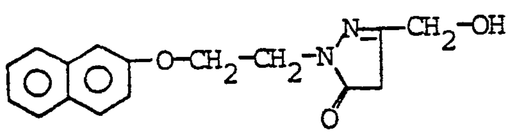 | $10^{-5}$<br>$5.10^{-6}$ | 80<br>61 |
| 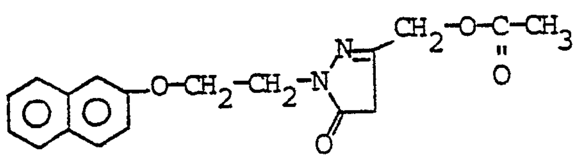 | $10^{-5}$<br>$5.10^{-6}$<br>$10^{-6}$ | 82<br>52<br>36 |
| 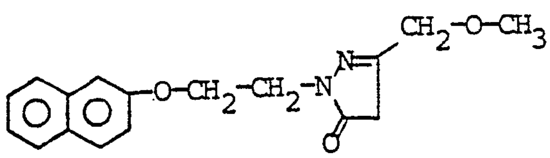 | $10^{-5}$<br>$5.10^{-6}$<br>$10^{-6}$ | 93<br>80<br>40 |
| 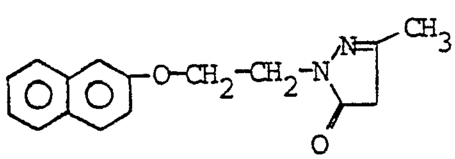<br>(Vergleich) | $10^{-5}$<br>$5.10^{-6}$<br>$10^{-6}$<br>$5.10^{-7}$ | 100<br>100<br>58<br>30 |

Beispiel 2

Spezifische Protacyclinsynthese-Stimulation

Die spezifische $PGI_2$-stimulierende Wirkung der Pyrazolonderivate wurde in vitro in einem Gemisch aus Mikrosomen von Schafssamenblasen (RSVM) und Rinderaorten (BAM) gezeigt (vgl. F. Cottee et al., Prostaglandins, 14, 413, 1977). $^3$H-Arachidonsäure wurde in Gegenwart von $3.10^{-5}$ g/ml des Pyrazolonderivats mit einem Gemisch aus RSVM und BAM 10 Min. bei 25°C inkubiert. Die Reaktion wurde durch Ansäuern auf pH 3,5 gestoppt. Die Fettsäuremetaboliten wurden mit Essigester extrahiert. Der Essigester wurde unter $N_2$ abgedampft und der Rückstand in $CH_3OH/CHCl_3$ (1:1) aufgenommen und auf DC-Plastikfolien aufgetragen. Die Trennung erfolgte mit einem Fließmittelgemisch Ethylacetat/Eisessig/Isooctan/$H_2O$ (110:20:50:10; organische Phase) (P. Needleman et al., The Journal of Clinical Investigation 1978, 61, 839-849). Die Verteilung der Radioaktivität wurde mittels eines Radioscanners gemessen.

Die nachfolgende Tabelle zeigt den Einfluß der Pyrazolonderivate ($10^{-4}$ g/ml) auf die Synthese verschiedener Prostaglandine in RSVM gegenüber dem Kontrollversuch ohne Wirkstoff:

Tabelle 2

| Prostaglandin | Erhöhung der Konzentration (in %) gegenüber der Kontrolle | |
|---|---|---|
| | Verbindung 1 | Verbindung 2 |
| 6-Keto-$PGF_{1\alpha}$ | 300 - 400 | 250 - 300 |
| $PGD_2$ | 0 | 0 |
| $PGE_2$ | 0 | 0 |
| $PGF_{2\alpha}$ | 0 | 0 |

Verbindung 1

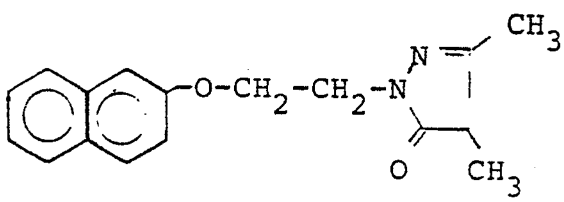

Verbindung 2

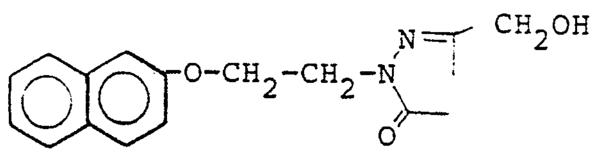

Beispiel 3

Leukozytenklebrigkeit

Die Zahl der Leukozyten, die ein Gefäß in einem bestimmten Abschnitt passieren, dient als einfaches Modell zur Messung der Klebrigkeit von Leukozyten (Bray, M.A. et al., Prostaglandins 22, 213 (1981)). Die Anzahl der gezählten Leukozyten wird umso größer, je geringer die Klebrigkeit dieser Zellen wird.

Die unten beschriebenen Pyrazolonderivate wurden in diesem Modell untersucht.

Männliche Syrische Goldhamster (80-100 g) wurden mit Nembutal (i.p.; 60 mg/kg) narkotisiert. Nach Einbinden eines PE 10 Katheters in die A. femoralis wird das Tier auf eine Präparierbühne nach Duling (MVR 5, 423 (1973)) gelegt und die rechte Backentasche durch Einführen eines Q-Tips herausgezogen. Sie wird vorsichtig in den dafür vorgesehenen Teil der Bühne gezogen, ausgespannt und fixiert.

Ab Beginn der Präparation wird die vorbereitete Backentasche mit 5 ml/Min. Superfusat überspült. Die Temperatur der Lösung beträgt 36°C. Unter Schonung der Gefäße wird die obere Gewebsschicht längs durchtrennt und zur Seite geklappt. Bei ca. 200-facher Vergrößerung wird eine Fläche von ca. 1 $cm^2$ von Bindegewebe vorsichtig freipräpariert. Das Tier wird mit der Präparierbühne auf den Objekttisch des Mikroskops gelegt. Ein Thermofühler wird in die linke Backentasche eingeführt. Die Körpertemperatur des Tieres wird so kontrolliert und mit Hilfe einer IR-Lampe (250 Watt) auf 36 ± 0.3°C gehalten.

Die Messung der Leukozytenklebrigkeit wird bei ca. 500-facher Vergrößerung vorgenommen. In dem freipräparierten Feld wird eine Venüle (30 - 40 µm Ø ) ausgewählt. In einem bestimmten Abschnitt dieses Gefäßes wird die Anzahl der pro Zeiteinheit (Min.) vorbeiwandernden Leukozyten gezählt.

Die i.a. Applikation der Pyrazolonderivate in 1 % wäßriger Tylose-Suspension führte zu folgenden Ergebnissen:

Tabelle 3

| Verbindung | Dosis (mg/kg) | Erhöhung der Leukozytenzahl (%) | Wirkdauer (Minuten) |
|---|---|---|---|
| 1 | o,1 | 75 | 30 |
| 2 | 1 | 25 | 30 |
| 3 | 1 | 200 | ≫60 |
| 4 | 0,1 | 35 | 40 |
| 5 | 1 | 200 | 30 - 50 |
| 6 | 1 | 30 | 20 - 30 |

Verbindung 1

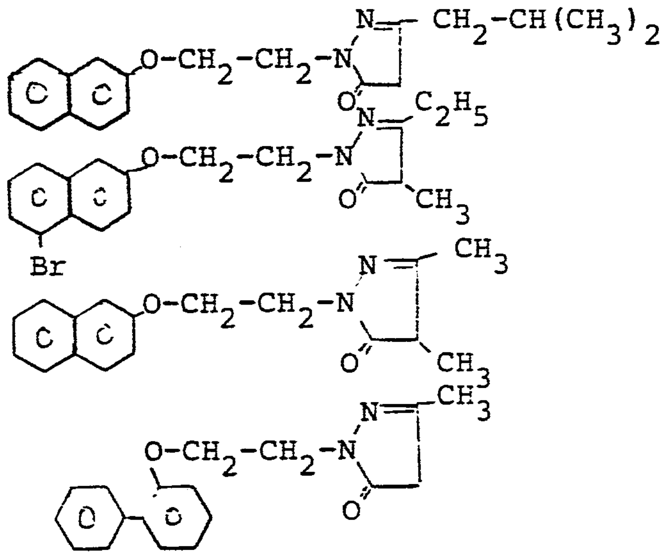

Verbindung 2

Verbindung 3

Verbindung 4

Verbindung 5

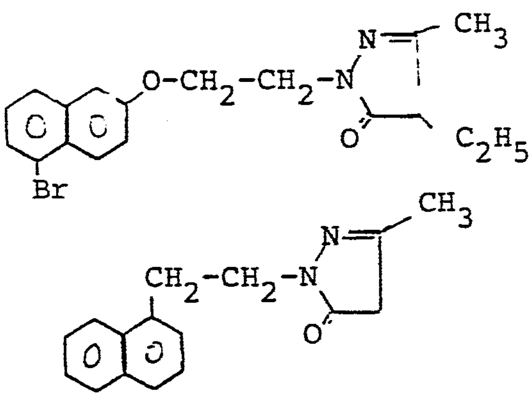

Verbindung 6

Beispiel 4

Wirkung der Pyrazolonderivate am nach Langendorff perfundierten Kaninchenherzen

Von den Kaninchen (bis ca. 2 kg) wurde das Herz unter Nembutal-Narkose entnommen und an der Aorta sowie an der A. pulmonalis kanüliert. Zur Messung der isovolumetrischen Kontraktionen wurde ein flüssigkeitsgefüllter Ballon aus Silikonkautschuk über den linken Vorhof in den linken Ventrikel eingeführt. Über eine Flüssigkeitsbrücke war der Ballon mit einem Flüssigkeitsaufnehmer (Statham P 23 Db) verbunden. Der Perfusionsdruck wurde mit einem Druckaufnehmer, der vor der Aortenkanüle des Herzens über ein T-Stück mit dem Perfusionssystem verbunden war, aufgenommen. Die Registrierung von Kontraktionskraft und Perfusionsdruck erfolgte mit dem Schnellschreiber Gould 2600 S. Die Herzen wurden elektrisch stimuliert mit der Frequenz 180/Min., Reizdauer 5 msec.

Die Perfusion des Herzens erfolgte mit Krebs-Henseleit-Lösung (KH-Lösung), die mit Carbogen (95 % $O_2$, 5 % $CO_2$) oder mit der Gasmischung 2 % $O_2$, 5,6 % $CO_2$, Rest $N_2$ begast wurde. Das Perfusionsvolumen betrug 20 ml/Min. das über eine Rollenpumpe (Fa. Desaga, 132100) eingestellt war. Die Zugabe des Pyrazolonderivats (in 1 % wäßriger Tylose-Suspension) und der Thrombozytensuspensionen (aus denselben Kaninchen, aus denen das Herz entnommen ist, gewonnen) erfolgte mit Hilfe von Infusionspumpen (Rollenpumpen, Fa. Braun-Melsungen), wobei die Lösung oder Suspension mit der Fließgeschwindigkeit von 0,2 bzw. 0,1 ml/Min. aus einer Infusionsspritze über einen Taigonschlauch und eine feine Kanüle kurz vor dem Herzen in den zuführenden Schlauch infundiert wurde.

Die Endkonzentration der Thrombozyten betrug 1 x $10^7$/ml KH-Lösung. Die aus dem Herzen (Kanüle in A. pulmonalis) fließende Lösung wurde periodisch unter Eiskühlung aufgefangen. Zur Messung der Konzentrationen von Thromboxan als $TXB_2$ und von Prostacyclin als 6-Keto-$PGF_{1\alpha}$, deren Bestimmung mit Hilfe von Radioimmunassays erfolgte, wurden aliquote Volumenanteile gefriergetrocknet und in einem Zehntel des ursprünglichen Volumens mit Wasser aufgenommen.

Die in den Versuchen gefundenen Werte des Perfusionsdruckes bei Gabe des Wirkstoffes im Vergleich zur Kontrolle sind aus der nachfolgenden Tabelle 4 ersichtlich:

Tabelle 4

Perfusionsdruck in % des Nullwertes

| | 1.Phase | 2.Phase | 3.Phase | 4.Phase | 5.Phase |
|---|---|---|---|---|---|
| Kontrollversuch | 100 | 155 | - | 83 | 214 |
| Verbindung 1 | 100 | 274 | 108 | 82 | 97 |
| Verbindung 2 | 100 | 176 | 107 | 83 | 103 |

1. Phase Vorphase; Begasung mit Carbogen
2. Phase Normoxie; Begasung mit Carbogen; Zusatz von $1.10^7$ Thrombozyten/ml; Dauer: 5 Minuten
3. Phase wie 2. Phase; Zusatz von $1.10^{-5}$ g/ml Pyrazolonderivat; Dauer: 5 Minuten
4. Phase wie 3. Phase, jedoch Begasung mit nur 2 % $O_2$ (Hypoxie); Dauer: 10 Minuten
5. Phase wie 3. Phase (Reoxygenierung).

Verbindung 1

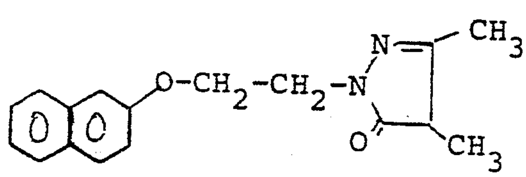

Verbindung 2

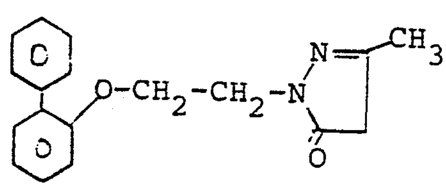

Die Ergebnisse zeigen bei Zusatz von Thrombozyten in die Perfusionslösung einen signifikanten Anstieg des Perfusionsdruckes im Vergleich zur Vorphase. Wird in der nachfolgenden Phase mit sauerstoffarmer Lösung perfundiert (2 % $O_2$), so fallen der Perfusionsdruck und die Kontraktionskraft stark ab. Bei anschließender Reoxygenierung (Carbogenbegasung) erholt sich die Kontraktionskraft auf annähernd gleiche Werte wie vor der Hypoxieperiode; der Perfusionsdruck steigt jedoch über das Druckniveau der ersten Carbogenphase hinaus an.

Überraschend ist, daß die erfindungsgemäß einzusetzenden Pyrazolonderivate den Anstieg des Perfusionsdruckes, der durch Zusatz von Thrombozyten zur Perfusionslösung in der ersten und zweiten Carbogenphase bewirkt wird, signifikant inhibieren. Besonders ausgeprägt zeigt sich diese Wirkung nach der Hypoxiephase in der Reoxygenierungsphase.

Beispiel 5

Wirkung der Pyrazolonderivate auf experimentellen Herzinfarkt des Hundes

Die Wirkungen verschiedener Pyrazolonderivate auf den experimentellen Herzinfarkt wurden im Hund nach akuter Unterbindung einer Koronararterie untersucht. Bei Pentobarbital-narkotisierten Hunden wurde die vordere absteigende linke Koronararterie (LAD) proximal langsam für 6 Stunden unterbunden. Die Arterie wurde nicht reperfundiert. Eine Stunde vor Ligatur wurde das Pyrazolonderivat in 1 %iger wäßriger Tylosesuspension intraduodenal als Bolus gegeben. In den Kontrollen wurde gleichermaßen vorgegangen, jedoch ohne Wirkstoffgabe. Die Infarktgröße und das Perfusionsgebiet der LAD wurden durch eine duale Perfusionsmethode mit Evans-Blau und Triphenyltetrazolium Chlorid dargestellt. Nach der graduellen, langsamen LAD-Unterbindung verkleinerte das Pyrazolonderivat den Infarkt beträchtlich. Dies war zu beobachten sowohl in Bezug auf das absolute Infarktgewicht als auch in Bezug auf die Infarktgröße relativ zum linken Ventrikelgewicht. Die Perfusionsgebiete der unterbundenen Arterien waren gleich. Initial sank als Folge der Koronarligatur der Blutdruck, was zur reflektorischen Herzfrequenzsteigerung führte. Sonst wurden keine durch Pyrazolonderivate verursachten, hämodynamischen Wirkungen gesehen. Die Verbindungen verminderten die Infarkt-bedingten Erhöhungen der ST-Segmente und der R-Zacke des peripheren EKG als Ischämiezeichen

was auf elektrophysiologische Wirkungen schließen ließ.
Literatur:
Lucchesi et al., J.Pharmacol.Exp. Ther. 199: 310, 1978;
Fiedler, Basic Res.Cardiol. 78: 266, 1983.

Die Ergebnisse sind in den nachstehenden Tabellen zusammengefaßt.

N Zahl der im jeweiligen Experiment untersuchten Hunde

$N_1$ Zahl der durch die Ligatur getöteten Hunde.

Verbindung 1:

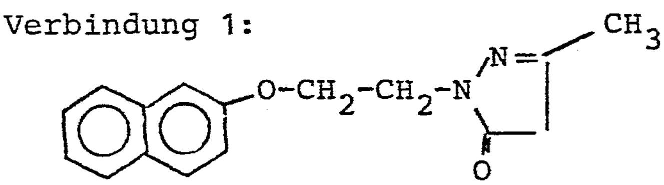

(Vergleich)

Verbindung 2:

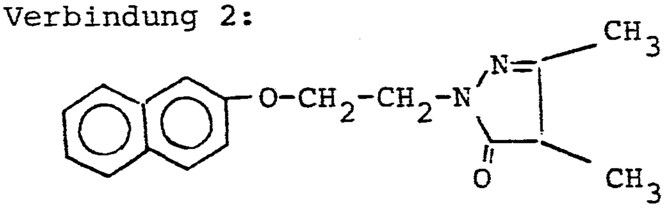

Verbindung 3:

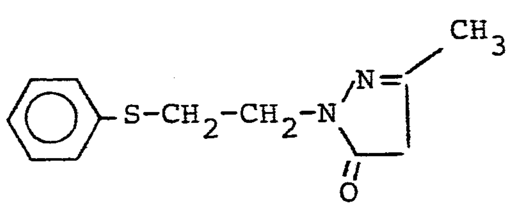

Tabelle 1

Mortalität nach 6 h LAD-Ligatur

| Verbindung | N | Dosis (mg/kg) | Mortalität ($N_1$) |
|---|---|---|---|
| 1 | 4 | 1 | 2 |
| | 4 | 3 | 0 |
| | 6 | 10 | 1 |
| 2 | 4 | 1 | 3 |
| | 4 | 3 | 0 |
| | 8 | 10 | 0 |
| 3 | 2 | 10 | 0 |

Tabelle 2

Zeit, nach welcher Exitus durch Kammerflimmern eintritt; insgesamt 4 h LAD-Ligatur

| Verbindung | N | Dosis (mg/kg) | $N_1$ | Exitus nach |
|---|---|---|---|---|
| | 5 | 10 | 2 | 14 min 23 sec.; 3 h 37 min |
| 1 | 4 | 3 | 2 | 25 min 40 sec.; 3 h 21 min |
| | 4 | 1 | 3 | 24 min; 35 min; 2 h 2 min. |
| | 8 | 10 | - | - |
| 2 | 4 | 3 | - | - |
| | 4 | 1 | 2 | 2 h 25 min; 3 h 45 min |
| | 3 | 0,5 | 1 | 2 h 16 min |

Tabelle 3

Größe des Infarkts nach 6 h LAD-Ligatur

| Dosis | N | Gewicht des linken Ventrikels (g) | Infarktgröße Gewicht (g) | Infarktgröße % des linken Ventrikels |
|---|---|---|---|---|
| Kontroll-gruppe | 8 | 97,3 ± 4,3 | 24,1±3,4 | 24,8±2,8 |
| 10 mg/kg Verbin-dung 2 | 8 | 95,2 ± 3,2 | 12,1±1,9 | 12,7±2,1 |

Patentansprüche

1) Verwendung von Verbindungen der allgemeinen Formel (I)

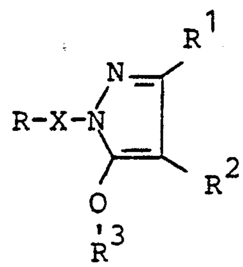

(I)

worin

R für einen Aryl- oder Heteroarylrest mit 6 bis 12 C-Atomen steht, der gegebenenfalls 1 bis 3 gleiche oder verschiedene Substituenten aus der Gruppe Halogen, Trifluormethyl, Alkyl, Alkenyl, Alkoxy, Alkylamino, Cyano, Trifluormethoxy, Nitro, Hydroxy, $SO_n$-Alkyl (n = 0 bis 2) oder $SO_n$-Trifluormethyl (n = 0 bis 2) enthält, wobei unter Alkyl, Alkenyl und Alkoxy jeweils Reste mit 1 bis 4 C-Atomen zu verstehen sind,

$R^1$ Wasserstoff oder einen Kohlenwasserstoffrest mit 1 bis 18 C-Atomen, welcher gegebenenfalls eine Hydroxy-, Ether-, Ester- oder Carboxylgruppe sowie gegebenenfalls 1 bis 3 Halogenatome enthält, darstellt,

$R^2$ die Bedeutung von $R^1$ hat oder für einen Rest

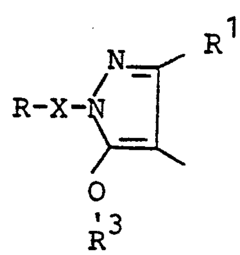

steht,

$R^3$ Wasserstoff, einen Acyl- oder Sulfonylrest mit jeweils 1 bis 15 C-Atomen darstellt und

X für eine der Gruppen
$-O-CH_2-CH_2-$, $-S-CH_2-CH_2-$, $-CH_2-CH_2-CH_2-$, $-CH_2-CH_2-$, $-CH_2-$ oder $-CH(CH_3)-$

steht, wobei das Sauerstoffatom an den Rest R gebunden ist, mit der Maßgabe, daß X nicht für $-O-CH_2-CH_2-$ steht, wenn R = ß-Naphthyl; $R^1$ = $CH_3$ und $R^2$ = $R^3$ = H;

- als Lipoxygenasehemmer, zur Verhütung und Behandlung von Ischämien und von Herzrhythmusstörungen, sowie von rheumatischen, allergischen und asthmatischen Erkrankungen, Oedemen, Lungenembolien, Schocklunge, pulmonaler Hypertension, Sauerstoffintoxikationen, Haut- und Augenentzündungen und Ulcerationen.

2) Verwendung nach Anspruch 1, dadurch gekennzeichnet, daß in Formel (I) R für einen gegebenenfalls durch 1 oder 2 Substituenten, insbesondere Cl oder Br, substituierten Naphthyl-, Diphenyl- oder Phenylrest steht.

3) Verwendung nach Anspruch 2, dadurch gekennzeichnet, daß R für einen gegebenenfalls substituierten Naphthylrest steht.

4) Verwendung nach Ansprüchen 1 bis 3, dadurch gekennzeichnet, daß in Formel (I) $R^1$ und $R^2$ für Wasserstoff oder einen Kohlenwasserstoffrest mit 1 bis 8 C-Atomen, welcher gegebenenfalls eine Hydroxyl-, Ether- oder Estergruppe aufweist, vorzugsweise eine gegebenenfalls verzweigte $C_1$-$C_4$-Alkylgruppe, stehen, wobei entweder $R^1$ oder $R^2 \neq H$ und $R^1$ und $R^2$ zusammen vorzugsweise 2 bis 7 C-Atome aufweisen.

5) Verwendung nach Ansprüchen 1 bis 4, dadurch gekennzeichnet, daß in Formel (I) $R^3$ für Wasserstoff oder einen aromatischen Acylrest mit 7 bis 13 C-Atomen, vorzugsweise für Wasserstoff steht.

6) Verwendung nach Anspruch 1 von 3,4-Dimethyl-1-/2-(2-naphthyloxy)-ethyl7-2-pyrazolin-5-on.

7) Arzneimittel gegen Haut- und Augenentzündungen und Schocklunge sowie lipoxygenasehemmendes, antirheumatisches, antiallergisches, antiasthmatisches, antiischämisches, antiarrhythmisches, antioedemisches, bronchodilatorisches und gastroprotektives Arzneimittel, dadurch gekennzeichnet, daß es eine therapeutisch wirksame Menge einer Verbindung gemäß Formel (I) von Anspruch 1 enthält.

8) Arzneimittel nach Anspruch 7, dadurch gekennzeichnet, daß es eine für die orale Verabreichung geeignete Form aufweist.

9) Arzneimittel nach Anspruch 7, dadurch gekennzeichnet, daß es eine für die parenterale Verabreichung geeignete Form aufweist.

10) Verfahren zur Herstellung eines Arzneimittels nach Ansprüchen 7 bis 9, dadurch gekennzeichnet, daß man eine therapeutisch wirksame Menge einer Verbindung gemäß Formel (I) mit einem pharmazeutisch unbedenklichen Verdünnungsmittel oder Trägermaterial vermischt und in eine geeignete Applikationsform überführt.